# EUROPEAN PATENT APPLICATION

(11) **EP 0 939 129 A1**
(43) Date of publication of application: **01.09.1999**
(21) Application number: 98200498.8
(22) Date of filing: 17.02.1998
(51) Int. Cl.: C12N 15/52, C12N 9/00, C12N 1/20, C12Q 1/68, G01N 33/53, A01G 7/00

(54) **Increase of nodule number and nitrogen fixation in leguminosae**

(30) Priority: 09.02.1998 EP 98200380
(71) Applicant: K.U. Leuven Research & Development, 3000 Leuven (BE)
(72) Inventor: Rosemeyer, Viola, 1300 Wavre (BE); Vanderleyden, Josef, 3001 Beverlee (BE)
(74) Representative: Van Someren, Petronella F. H. M.

(57) **Abstract**

The present invention relates to a new mutant Rhizobium etli CNPAF 512 strain, having an inactivated raiI gene. Inactivation of the raiI gene leads to increased nodule number in beans upon inoculation thereof with the mutant strain. Based on this specific strain other strains can be mutated in the similar gene(s) in order to enable nodule increase and nitrogen fixation in other Leguminosae species.

## Description

The present invention relates to a new mutant Rhizobium strain that is deficient in a gene involved in autoinducer synthesis. The invention also relates to a method of increasing the nitrogen fixation in Leguminosae and a method of providing a Rhizobium strain having a mutation in a gene involved in autoinducer synthesis and the product of this method.

Autoinduction is a highly conserved mechanism of differential gene expression in many Gram-negative bacteria. The key trigger of this system is the concentration of small diffusible molecules, termed autoinducers in respect to their biological activity. All autoinducers so far identified are N-acyl homoserine lactones (AHLs). They are synthesized by an autoinducer synthase, the product of a luxI-homologous gene, and are thought to bind to a protein belonging to the LuxR-family of transcriptional activators. This autoinducer-protein complex activates the expression of defined genes or sets of genes. As this gene activation occurs only when a required threshold concentration of AHLs is attained, the onset of specific genes is dependent on the cell density of bacteria. Consequently, autoinduction allows bacteria to monitor their own population density and to discriminate between high and low cell density. It can also be understood as a cell-cell communication system.

The physiological processes regulated by autoinduction are diverse, as exemplified by the following systems: bioluminescence in Vibrio fischeri, plasmid conjugal transfer in Agrobacterium tumefaciens, antibiotic production in Erwinia carotovora, and synthesis of exoenzymes in plant and animal pathogens like E. carotovora and Pseudomonas aeruginosa. In Escherichia coli, a LuxR-homologue is involved in the regulation of cell division.

Rhizobium etli CNPAF512 (formerly classified as R. leguminosarum bv. phaseoli CNPAF512) forms nitrogen-fixing nodules on the roots of common bean. Within this structure bacteria are densely packed and differentiate into their symbiotic state, the bacteroids, able to reduce atmospheric dinitrogen into ammonia. In view of the high cell density during bacteria-plant interaction, autoinduction may be involved at some stage of symbiosis.

Since the nitrogen-fixation that occurs in the nodules is important for plants, it is always desirable to be able to increase the nitrogen-fixing capacity per plant.

In the research that led to the present invention a Rhizobium etli CNPAF512 gene has now been identified by which the Rhizobium strain containing the gene is able to control the number of nodules formed per plant. Based on this information a mutant could be developed in which this particular gene is inactivated and inactivation of this gene led to a significant increase in the number of nodules formed per plant.

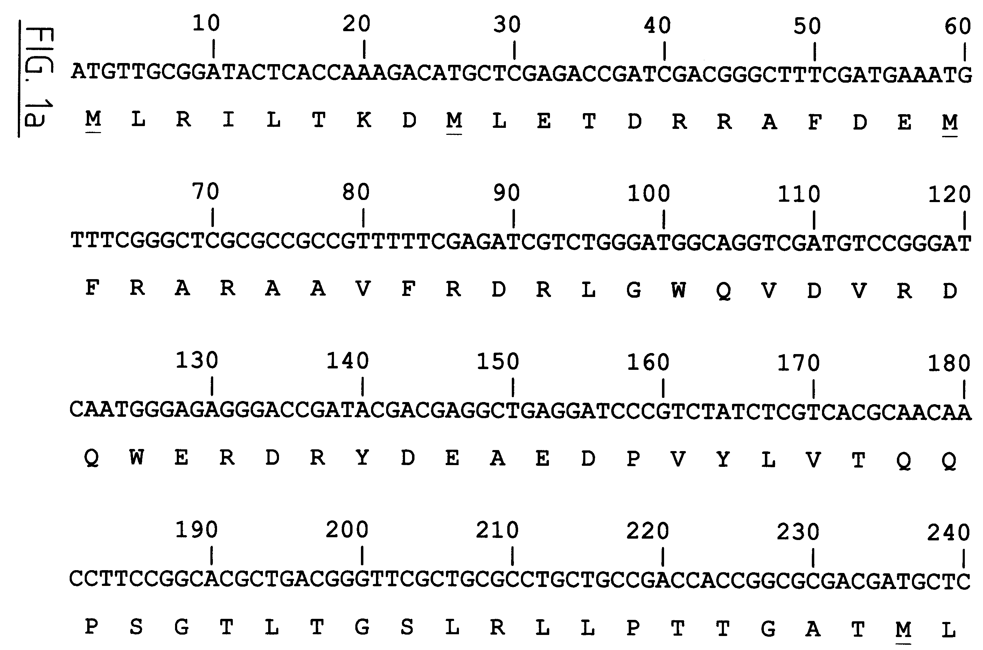

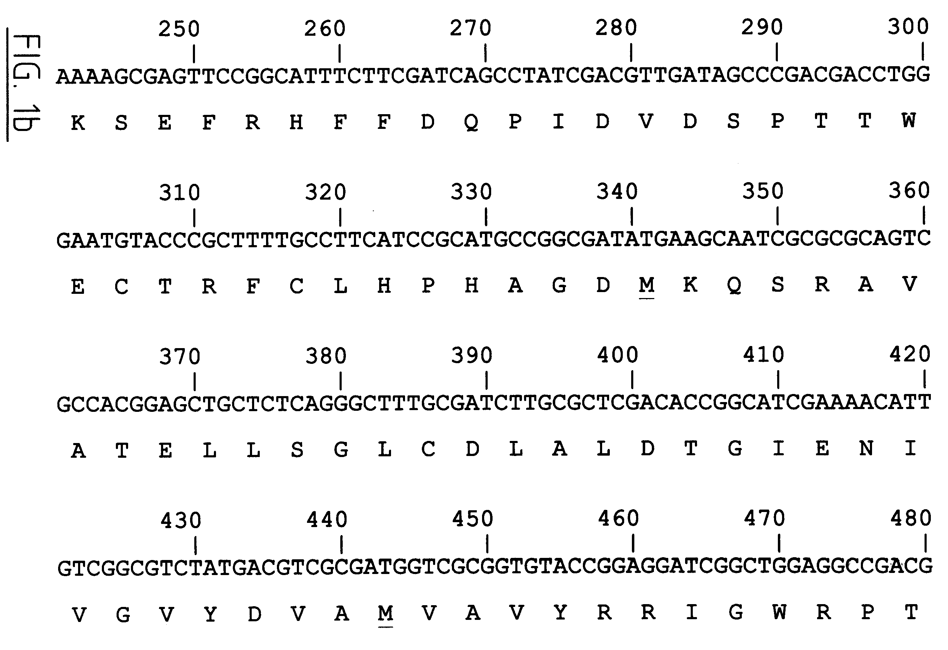

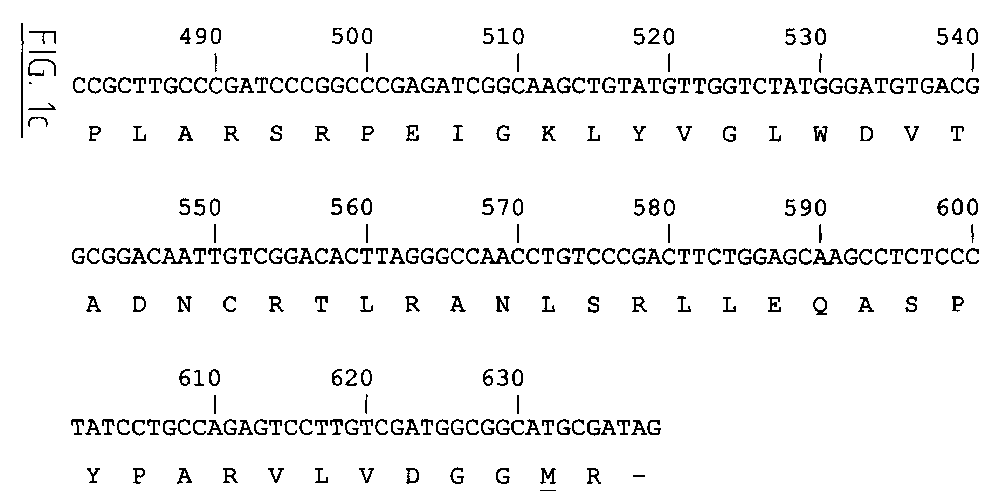

The invention thus relates to a new mutant Rhizobium etli CNPAF512 strain, in which the biological function of the raiI gene is inactivated. More in particular the invention relates to a mutant Rhizobium etli CNPAF512 strain having a gusA-Km insertion in its raiI gene. More in particular, the invention provides a mutant Rhizobium etli CNPAF512 strain, herein designated as FAJ1328 and having a gusA-Km insertion in the XhoI restriction site of the raiI gene. The nucleotide sequence of the raiI gene is given in figure 1.

Based on the identification of the raiI gene in Rhizobium etli CNPAF512 it became possible according to the invention to create other mutant rhizobia strains by:
a) identifying a gene that is at least 40% similar with the raiI gene of Rhizobium etli CNPAF512 strain in any rhizobia strain; and
b) inactivating the biological function of the gene similar to raiI. In this application the term 'similar' intends to encompass both situations in which sequence homology exists and situations wherein a similar biological function (autoinduction) exists

Inactivation of the biological function can be achieved in various ways.

First, the gene similar to raiI can be mutated by either an insertion of any DNA sequence that is capable of disrupting the reading frame or by deletion of a part of the coding sequence, for example also leading to disruption of the reading frame. Mutations that lead to an internal stop codon can also be used to inactivate the biological function.

Second, the biological function of the raiI gene or other genes encoding autoinducer synthases can be inactivated by interfering with their transcription regulation factors. This interference leads to a complete or partial lack of transcription of the raiI gene.

"Biological function" is to be understood as comprising both the direct biological function of the gene, i.e. synthesis of the gene product encoded by the gene and the effect thereof, or its indirect biological function, e.g. synthesis of other molecules like autoinducers, the synthesis of which is dependent on the expression of the raiI gene, and the effect of the synthesis of those molecules. For example, for Rhizobium etli CNPAF512, one of the ultimate biological functions of the non-mutated raiI gene is limitation of the number of nodules formed upon infection of a species of the Leguminosae.

The rhizobia component of the nodules of Leguminosae is specific at least to the genus of the Leguminosae family members. A skilled person will very well be capable of selecting a suitable rhizobia species for the plant in which the nodule number should be increased. Starting from this rhizobia species mutants can be created in which the biological function of the raiI (homologous) gene or other genes encoding autoinducer synthases is inactivated. The rhizobia encompass inter alia the genus Rhizobium and the genus Bradyrhizobium.

The invention further relates to rhizobia strains being deficient in the biological function of their raiI gene or raiI homologous gene or other genes encoding an autoinducer synthases, obtainable by the method as described above.

According to a further aspect thereof, the invention relates to such mutant rhizobia strains for use in increasing the number of nodules in their corresponding host plants and to a method for increasing the nitrogen fixation in Leguminosae, comprising inoculating Leguminosae plants with a mutant strain and providing circumstances suitable for the rhizobia strain to induce nodules.

In a first preferred embodiment of the invention the Leguminosae plant is a bean plant and the rhizobia strain is a strain modulating bean such as Rhizobium leguminosarum biovar phaseoli.

In a second preferred embodiment the Leguminosae plant is a Phaseolus plant and the rhizobia strain is Rhizobium etli CNPAF512.

In a third preferred embodiment the Leguminosae plant is a Glycine max plant and the rhizobia strain is soy-bean nodulating strain selected from Bradyrhizobium japonicum CB1809 and Bradyrhizobium elkani BR29W.

According to still a further aspect of the invention there is provided the raiI gene of Rhizobium etli CNPAF512 in substantially isolated form, comprising a coding nucleotide sequence that is identical to the coding sequence of the nucleotide sequence depicted in figure 1 or at least 90% similar to this sequence, or the complementary sequence of either of these. This raiI gene can be used as a probe for screening other rhizobia strains for the presence of a gene that is at least 40% similar to raiI of Rhizobium etli CNPAF512.

The present invention will be further illustrated by the following examples. First it is demonstrated how the presence of the raiI gene was detected through its biological function. Then the relevant genes are isolated (Example 2). Creation of the mutant is described in Example 3, whereas Example 4 illustrates the effect of the mutation on the biological function. Example 5 shows the detection of raiI similar genes in Bradyrhizobium. Example 6 shows mutagenesis of genes encoding autoinducer synthase in other rhizobia species. Example 7 shows the use of raiI as a probe for screening other rhizobia strains.

In the examples reference is made to the following figures:
**Fig. 1:** Nucleotide sequence of the R.etli CNPAF512 raiI gene and deduced amino acid sequence.

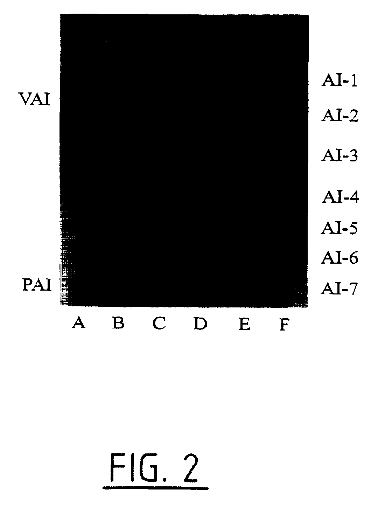

**Fig. 2:** Autoinducers produced by (A) Vibrio fischeri (VAI) and Pseudomonas aeruginosa (PAI; purified autoinducers), (B) E. coli DH5α, (C) E. coli DH5α containing the R. etli CNPAF512 raiI and raiR in pUC18 (FAJ1323), (D) R. etli CNPAF512 wild type, (E) the R. etli CNPAF512 mutant with disrupted raiI (FAJ1328), and (F) the R. etli CNPAF512 mutant with disrupted raiR (FAJ1329). Ethyl acetate extracts (B-E) were spotted on a C₁₈ reversed phase thin layer chromatography plate. 60% methanol was used as liquid phase. Molecules with autoinducer activity were visualized with a soft agar overlayer containing X-Gal and A. tumefaciens indicator cells.

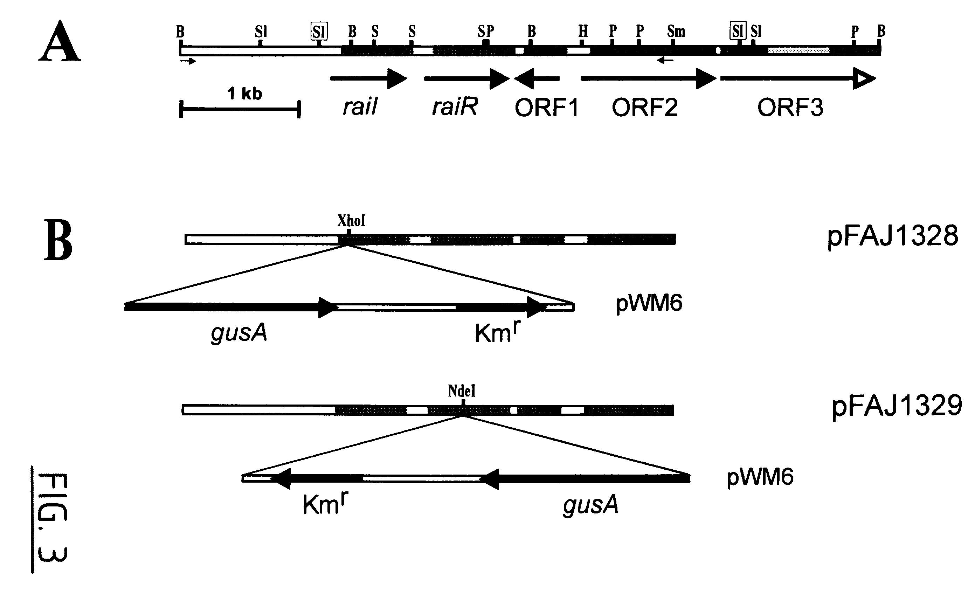

**Fig. 3:** (A) Physical and genetic map of the region containing raiI and raiR from R. etli CNPAF512. Grey shading illustrates coding regions. Arrows indicate directions of transcriptions. The open arrow represents a beginning open reading frame. Light grey color illustrates a part of ORF3 that has not been sequenced. The boxed SalI sites have been used for construction of pFAJ1323. The small arrows indicate the primers with NotI sites used for amplification of a 4 kb fragment. Restriction sites: B, BamHI; H, HindIII; P, PstI; S, SphI; Sl, SalI; Sm, SmaI. (B) Construction of the raiI (FAJ1328) and raiR (FAJ1329) mutant. The PCR product was cloned into pCR™2.1 TA, excised with NotI and inserted into pJQ200ucl. A cassette with a promoterless gusA and kanamycin resistance from pWM6 was introduced in the XhoI (raiI mutant) or NdeI (raiR mutant) site by blunt end ligation.

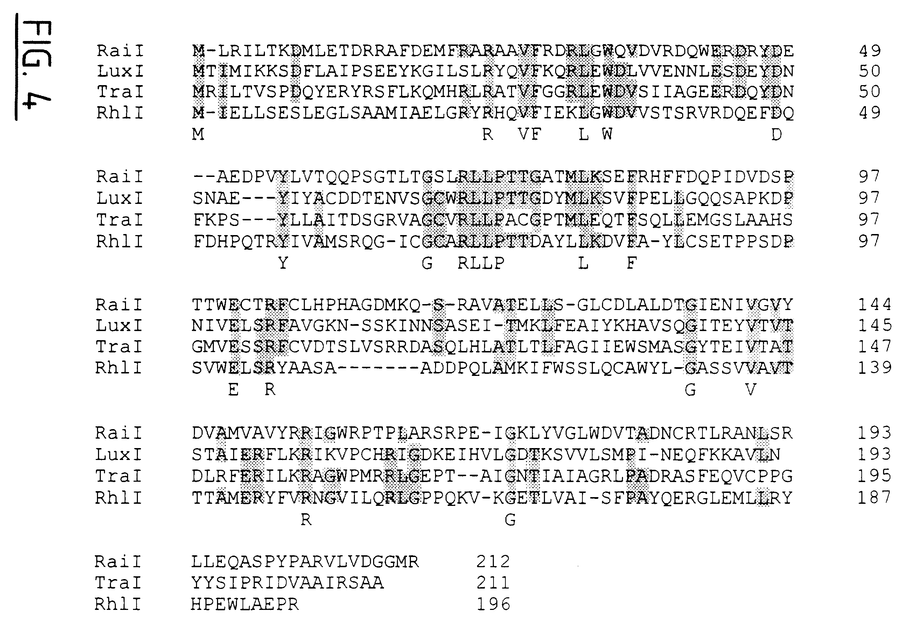

**Fig. 4:** Alignments of members of the LuxI family. A grey background indicates amino acid residues that are identical in at least three aligned sequences. Amino acid residues that are conserved in all the homologues aligned are shown below the sequences. The sequences for RhlI (Accession No. U40458) and RaiI (Accession No. U92712) are in the GenBank Sequence Data Library, LuxI (Accession No. P12747) and TraI (Accession No. P33907) are in the Swiss-Prot Protein Sequence Database.

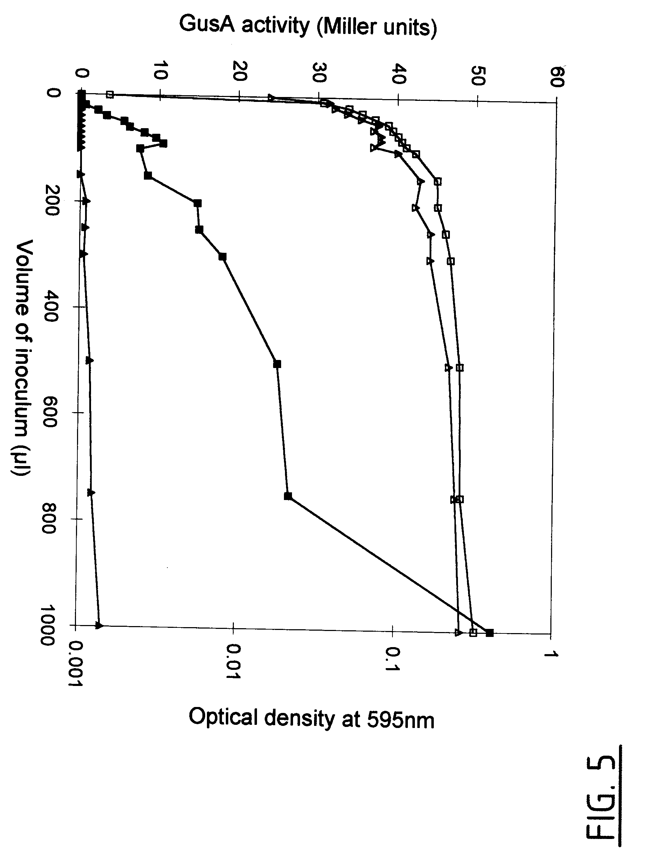

**Fig. 5:** Comparison of raiI expression in a wild type and raiI mutant background in dependence of cell density. Expression of the raiI::gusA fusions were monitored using p-nitrophenyl-β-D-glucuronide as substrate. Values of optical density and Miller units were based on measurement in microtiter plates. Curves show the averages of three separate experiments. Rectangles represent CNPAF512::pFAJ1328 (rail::gusA in wild type background), triangles FAJ1328 (rail knockout mutant), open symbols the optical density at 595 nm, and closed symbols GusA activity in Miller units.

### EXAMPLES

### BACTERIAL STRAINS, PLASMIDS, MEDIA AND CULTURE CONDITIONS

Bacterial strains and plasmids used in these examples are listed in Table 1.

Antibiotic concentrations were as follows: nalidixic acid, 30 µg/ml; ampicillin, 100 µg/ml; kanamycin, 25 µg/ml; neomycin, 60 µg/ml; tetracycline, 10 µg/ml; gentamicin, 25 µg/ml; carbenicillin, 100 µg/ml; rifampicin, 100 µg/ml.

Escherichia coli DH5α was grown in LB medium (Sambrook et al., Molecular Cloning - A laboratory manual. Cold Spring Harbor Laboratory Press, Cold Spring Harbor (1989)) at 37°C, Rhizobium etli CNPAF512 in TY medium (Beringer, J. Gen. Microbiol. 120: 421-429 (1974)) and A. tumefaciens NT1 (pJM749, pSVB33) in AB medium (Chilton et al., Proc. Natl. Acad. Sci. USA 71: 3672-2676 (1974)) at 28°C.

### EXAMPLE 1

### Extraction and detection of autoinducers

Rhizobium etli CNPAF512 was grown for 48 h and E. coli for 24 h in 500 ml medium to stationary phase. After centrifugation, the supernatant was extracted twice with an equal volume of ethyl acetate containing 1.5 ml acetic acid per litre. The extract was evaporated to dryness by vacuum rotation at 42°C and redissolved in a small volume of ethyl acetate. 1 µl of this suspension was spotted on a C₁₈ reversed phase thin layer chromatography (TLC) plate (RP-18F_{254S}, Merck), which was then developed with 60% methanol. The air-dried plate was overlaid with AB soft agar (Chilton et al., supra) containing 5-bromo-4-chloro-3-indolyl-β-galactopyranoside (X-Gal) and Agrobacterium tumefaciens NT1 (pJM749, pSVB33) indicator cells (Piper et al., Nature 362: 448-450 (1993), Shaw et al., Proc. Natl. Acad. Sci. USA 94: 6036-6041 (1997)).

This strain contains a Tn3HoHo1-generated lacZ fusion to a tra gene, the expression of which is dependent on TraR and autoinducer. As the clone lacks the Ti plasmid, it does not produce an autoinducer detectable with this system. Consequently, the lacZ reporter fusion is only expressed upon exogenous supply of autoinducer molecules (Piper et al., supra).

Incubation of the TLC plates at 28°C allowed the visualization of compounds activating the A. tumefaciens tra system expression. For comparison, purified autoinducer molecules from V. fischeri (VAI, N-(3-oxohexanoyl)-L-homoserine lactone) and from P. aeruginosa (PAI, N-(3-oxododecanoyl)-L-homoserine lactone) were used (E.P. Greenberg).

For fractionation, the ethyl acetate extract has been diluted 1:10 with 30% methanol in water, loaded on a C₁₈ reversed phase high performance liquid chromatography column (Bondclone 10 C18, Phenomenex) and eluted isocratically with 30% methanol. The fractions were tested for activation of the A. tumefaciens tra system and for bacteriostatic activity towards the sensitive strain R. leguminosarum bv. viciae 248 as described by Schripsema et al. (J. Bacteriol. 178: 366-371 (1996)).

After Rhizobium etli CNPAF512 was grown in 500 ml TY medium for 48h to stationary phase, the ethyl acetate extract of the cell-free spent medium was analyzed on a TLC plate for compounds activating the A. tumefaciens tra system expression (figure 2D). Seven distinct spots could be detected, indicating, that there are at least seven different autoinducer types present in R. etli CNPAF512. The existence of seven autoinducers in one species is described here for Rhizobium etli CNPAF512 for the first time.

Purified autoinducers from P. aeruginosa (PAI, N-(3-oxododecanoyl)-L-homoserine lactone) and V. fischeri (VAI, N-(3-oxohexanoyl)-L-homoserine lactone) were used for comparison (figure 2A). VAI migrated between AI-1 and AI-2 and PAI exhibited similar chromatographic features as AI-7.

The R. etli CNPAF512 ethyl acetate extract has been fractionated on a C₁₈ reversed phase high performance liquid chromatography column. Assaying the fractions for tra activation confirmed the presence of at least seven different autoinducer molecules as expected from the results of the TLC plates (data not shown).

After having demonstrated the presence of seven autoinducers in Rhizobium etli CNPAF512, corresponding genes could be identified as described in Example 2.

### EXAMPLE 2

### Identification of autoinducer encoding genes

Screening of a 5000 clones comprising R. etli CNPAF512 gene library in Escherichia coli HB101 was performed in microtiter plates. Each 100 µl of AB medium supplemented with 0.003% leucine, 0.023% proline, 0.004% thymine, and 0.0017% thiamine, containing A. tumefaciens indicator cells and X-Gal was pipetted into the wells of a microtiter plate. The clones of the gene library were added, and the plates incubated at 28°C while shaking.

After 12 h, eight wells were blue. Restriction analysis of the corresponding plasmids and hybridization with a V. fischeri luxI probe from pHV200 (Gray & Greenberg, J. Bacteriol. 174: 4384-4390 (1992)) revealed that they all contained an identical 3.8 kb SalI-fragment. E. coli DH5α containing this SalI-fragment in the pUC18 vector (FAJ1323) could still induce the A. tumefaciens test system to the same extent as the positive E. coli HB101 clones from the library.

Sequencing of pFAJ1323 revealed two open reading frames, the deduced amino acid sequences of which showed similarity to the autoinducer synthases of the LuxI family and to transcriptional activators of the LuxR family. Downstream of these genes are two complete open reading frames and the beginning of a third. The deduced ORF1 protein shows homology to members of the Lrp family of transcriptional regulators (Platko et al., J. Bacteriol. 172: 4563-4570 (1990)). The deduced ORF2 protein shows homology to a catabolic alanine racemase (DadX) and the deduced ORF3 protein shows homology to the small subunit of D-amino acid dehydrogenase (DadA) from E. coli (Lobocka et al., J. Bacteriol. 176: 1500-1510 (1994)). The racemase converts L-alanine to the D isomer, which is then oxidatively deaminated by the D-amino acid dehydrogenase to pyruvate and ammonia (Franklin & Venables, Mol. Gen. Genet. 149: 229-237 (1976)).

Figure 3A shows the physical map of this region, including the two BamHI fragments, that overlap the 3.8 kb SalI fragment.

The luxI homologous gene, termed raiI (Rhizobium autoinducer synthase), consists of a 639 bp open reading frame that possibly codes for an autoinducer synthase with 212 amino acids (figure 1). Sequence similarities of the deduced amino acid sequence with TraI, RhlI and LuxI are relatively low (Table 2). Highest similarities are clustered in the N-terminal region, especially from residue 22 to 36 and from 67 to 84 (referring to RaiI), which is a characteristic feature of LuxI homologues described so far (figure 4).

Amino acid residues found to be conserved in 12 LuxI homologues are also present in RaiI (Arg-24, Phe-28, Trp-34, Asp-48, Arg-70, Arg-104). However, instead of a cysteine at position 68 of LuxI, which is thought to be the active site of the autoinducer synthase, RaiI contains a serine.

raiI is followed by the luxR homologue raiR, separated by 144 basepairs. raiR consists of a 729 bp open reading frame and codes for a protein of 242 amino acids. Identities and similarities of RaiR with RhlR, TraR, and RhiR are given in Table 2. The LuxR-related transcriptional activators consist of an N-terminal regulator- and a C-terminal activator domain. Despite of the overall low similarity, the regions for autoinducer binding and the helix-turn-helix motif for DNA binding are conserved.

The organization of raiI and raiR differs from other species, as both are transcribed unidirectionally and the luxI homologue precedes the luxR homologue. In P. aeruginosa the gene order of the two autoinduction systems (las and rhl) is inversed, in A. tumefaciens traI and traR are not clustered. In all other identified autoinduction systems, both genes are either convergently or divergently transcribed.

The pattern of autoinducers produced by FAJ1323 (figure 2B) indicates that RaiI catalyses directly the synthesis of AI-2, AI-3, AI-4, and AI-5. Comparison with the autoinducers synthesized by an raiI mutant (figure 2E, see below) indicates, that the spots of AI-3 and AI-5 consist of comigrating molecules. Thus, AI-3 and AI-5 produced by FAJ1323 could be synthesized by RaiI, and the corresponding autoinducers produced by the raiI mutant by other autoinducer synthase(s) than RaiI.

The 3.8 kb SalI fragment then was inserted downstream of the lac promoter into the pUC18 vector, which is induced in the presence of isopropyl-β-D-thiogalactopyranoside. However, the synthesis of AI-3 and AI-5 occurs also in the absence of the inducer. It can therefore be concluded that the cloned fragment contained all necessary promoter elements.

Hybridization of total R. etli CNPAF512 DNA in a Southern blot with a raiI probe at low stringency gave rise to only one band.

### EXAMPLE 3

### Creation of mutants

A 4 kb region from pFAJ1322 (cf. description of figure 3) comprising raiI, raiR and ORF1, has been amplified by PCR. NotI sites were introduced by designing appropriate PCR primers (5'-CGCGCGGCCGCCATAGCCATCGCTGGTGATGTTGC-3' and 5'-CGCGCGGCCGCATGATAGGCATCGCCGAGAAAGAGG-3'). The product was cloned into the pCR™2.1 TA cloning vector (pFAJ1326). For verification, the terminal regions were sequenced. Subsequently, the fragment was excised with NotI and ligated into pJQ200uc1 (pFAJ1327; Quandt & Hynes, Gene 127:15-21 (1993)).

For mutagenesis of raiI, this construct was linearized with XhoI, allowing the insertion of a promoterless glucuronidase (gusA) gene coupled to a kanamycin resistance gene from pWM6 (Metcalf & Wanner, Gene 129: 17-25 (1993)) at 28 bp downstream of the predicted raiI translation start, resulting in a non-polar mutation (pFAJ1328; figure 3A).

The same cassette was inserted in inversed orientation into raiR via a unique NdeI site, 126 basepairs downstream of the predicted translation start (pFAJ1329; figure 3B). These plasmids were conjugated into R. etli CNPAF512 with the helper E. coli HB101/pRK2013, obtaining cis merodiploid recombinants with a raiI::gusA fusion (CNPAF512::pFAJ1328) or a gusA-Km insertion in raiR. Selection for double homologous recombination using the sacRB-based positive selection system on sucrose (Michiels, Dissertationes De Agricultura, 244 (1993)) led to a raiI mutant with a raiI::gusA fusion (FAJ1328) or a raiR mutant (FAJ1329), respectively. The genotypes of the mutants were verified by hybridization of a raiI-raiR probe and a probe of the pWM6 cassette to the same R. etli EcoRI fragment.

In order to examine the phenotypic relevance of raiI and raiR in R. etli, spent culture supernatants of the mutants FAJ1328 and FAJ1329 were extracted and analyzed on TLC plates. The chromatogram revealed, that the raiI mutant does not produce AI-1, AI-2, AI-4, and AI-6 in amounts detectable with the assay used (figure 2E). AI-3 and AI-5 are produced in a significantly lower amount than in the wild type. The absence of AI-2 and AI-4 confirmed that raiI is directly responsible for the synthesis of these two molecules. The failure of detection of AI-1 and AI-6 suggests, that their synthesis by autoinducer synthase(s) other than RaiI is dependent on autoinducers made by RaiI. In the raiR mutant, four molecules could be detected (figure 2F) : AI-7 and AI-5 in about the same concentration as in the raiI mutant, AI-3 in higher concentration as in the raiI mutant, and AI-4, which could not be detected in the raiI mutant. AI-1, AI-2, and AI-6 could not be detected, indicating that RaiR is necessary for their synthesis.

raiI expression in a wild type-(CNPAF512::pFAJ1328) and a mutant (FAJ1328) background was examined quantitatively in a cell density dependent way. As shown in figure 5, expression of raiI in a wild type background increased with cell density. In the mutant, raiI expression was nearly negligible, indicating that at least one of the autoinducers AI-1, AI-2, AI-4, or AI-6 is necessary for direct or indirect activation of raiI. raiI expression in FAJ1328 was restored in filter sterilized spent medium from R. etli CNPAF512. At low cell densities, expression levels in these conditions exceeded those of the wild type in fresh medium (data not shown).

### EXAMPLE 4

### Effect of the raiI mutation on nodule formation

Surface-sterilized and germinated bean seedlings (Van Rijn et al., J. Bacteriol. 175: 438-447 (1993)) were planted under sterile conditions in 0.5 times Jenssen medium (Vincent. A manual for the practical study of root-nodule bacteria. Blackwell Scientific Publications, Oxford/Edinburgh, UK (1970)) and subsequently inoculated with 200 µl of stationary phase bacterial cultures. The plants were grown at 26°C for 18 days with 12 h day length. Nitrogen fixation was measured in terms of acetylene reduction by gas chromatography (5890A, Hewlett Packard).

Phenotypic relevance of the rai genes for symbiosis with beans and for nitrogen fixation was examined. Germinated bean seedlings were planted under sterile conditions and subsequently inoculated with 200 µl of dense cultures (OD>1) of R. etli CNPAF512, FAJ1328 (raiI mutant), FAJ1329 (raiR mutant), or CNPAF512::pFAJ1334 (merodiploid in respect to raiI and raiR). The plants were grown at 26°C for 18 days. While no significant difference in delay of the appearance of the first nodules and dry weight of the shoot was observed (Table 3), the number of nodules per plant inoculated with the raiI mutant, FAJ1328, was about twice as high as for the plants inoculated with the wild type. The raiR mutant, FAJ1329, did not differ significantly from the wild type. Inoculation with the merodiploid CNPAF512::pFAJ1334 resulted in a significantly lower acetylene reduction per plant and a reduced nodule number, although a relatively high variation is observed. Values of nodule dry weight and of nitrogen fixation per plant illustrate this observation. However, nitrogen fixing activity in terms of acetylene reduction per nodule remained unchanged. It is therefore concluded that raiI, but not raiR, is involved in the restriction of nodule number, whereas nitrogen fixing activity is not affected. It is further concluded by comparison of the patterns of autoinducers produced by the raiI- and raiR mutant (figure 2E and F), that the molecules representing AI-3, which is synthesized at wild type levels in the raiR mutant but only present in a lower amount in the raiI mutant, or/and AI-4, which could not be detected in the raiI mutant, are involved in the regulation of nodule number.

### EXAMPLE 5

### Detection of autoinducers and the genes encoding autoinducer synthases in Bradyrhizobium

B. japonicum CB1809 and B. elkani BR29W were grown for 72 h in PSY medium (per litre: 3 g peptone, 1 g yeast extract, 0.3 g KH₂PO₄, 0.3 g Na₂HPO₄, 0.01 g MgSO₄, 0.01 g CaCl₂, 0.01 g H₃BO₃, 1 mg ZnSO₄ 7H₂O, 0.5 mg CuSO₄ 5H₂O, 0.1 mg MnCl₂ 4H₂O, 0.1 mg Na₂MoO₄ 4H₂O, 0.16 mg FeCl₃ H₂O) to stationary phase. After centrifugation, the supernatant was extracted twice with acidified ethyl acetate (1.5 ml acetic acid per litre). The extract was evaporated to dryness by vacuum rotation at 42°C and then dissolved in a small volume of acidified ethyl acetate. 1 µl of this suspension was spotted on a C₁₈ reversed phase thin layer chromatography (TLC) plate (RP-18F_{254S}, Merck), which was then developed with 60% methanol. The air-dried plate was overlaid with AB soft agar (Chilton et al., supra), containing 5-bromo-4-chloro-3-indolyl-β-galactopyranoside (X-Gal) and Agrobacterium tumefaciens NT1 (pJM749, pSVB33) indicator cells (Piper et al., supra, Shaw et al., supra). Incubation of the TLC plate at 28°C allowed the visualization of compounds activating the A. tumefaciens tra system expression. For comparison, extract from R. etli CNPAF512 was used.

For B. japonicum CB1809, three distinct spots could be detected, comigrating with AI-3, AI-4 and AI-5 from R. etli CNPAF512. The B. elkani BR29W a spot comigrating with AI-3 of R. etli CNPAF512 could be detected. For R. etli CNPAF512, it has been shown that AI-3 and/or AI-4 are crucial for the regulation of nodule number during symbiosis with its host plant. The presence of autoinducers with identical chromatographic features in both Bradyrhizobium strains show the presence of the same or an analogous regulation system of nodule number in the symbiosis between Bradyrhizobium and its host Glycine max.

The above described assay is used to screen a genomic library of Bradyrhizobium in order to find the gene responsible for the synthesis of autoinducer molecules.

### EXAMPLE 6

### Mutagenesis of genes encoding autoinducer synthases in other rhizobia species

pFAJ1328, a derivative of the suicide vector pJQ200uc1 (Quandt and Hynes, Gene **127:**15-21 (1993)) containing raiI with a gusA-Km^{r} insertion, raiR and ORF1, has been conjugated to either B. japonicum CB1809 or B. elkani BR29W with the helper E. coli HB101/pRK2073 or E. coli HB101/pRK2013, respectively. After selection using appropriate antibiotics (Ap¹⁰⁰, Cm³⁰, Tc¹⁵⁰, Km¹⁵⁰ for B. japonicum, Ap¹⁰⁰, Cm³⁰, Km¹⁵⁰ for B. elkani), mutants containing the disrupted raiI gene have been obtained.

Verification is performed by Southern hybridization. In order to knock out the Bradyrhizobium genes similar to raiI, selection for double homologous recombinants is carried out.

### EXAMPLE 7

### Use of raiI as a probe for screening other rhizobia strains

The 302 bp SphI fragment from pFAJ1323, containing the region from nucleotide 331 through nucleotide 632 of the raiI gene (see figure 1) has been used as hybridization probe for screening other rhizobia strains. Total DNA from R. leguminosarum biovar trifolii ANU843 and R. leguminosarum biovar trifolii ATCC 14483 (both can nodulate Trifolium) has been isolated and digested with EcoRI. The fragments have been separated by gel electrophoresis and were subsequently transferred to a nylon membrane. Hybridization was carried out under high stringency (5xSSC, 60°C). Detection showed that genes similar to raiI are present in the strains tested.

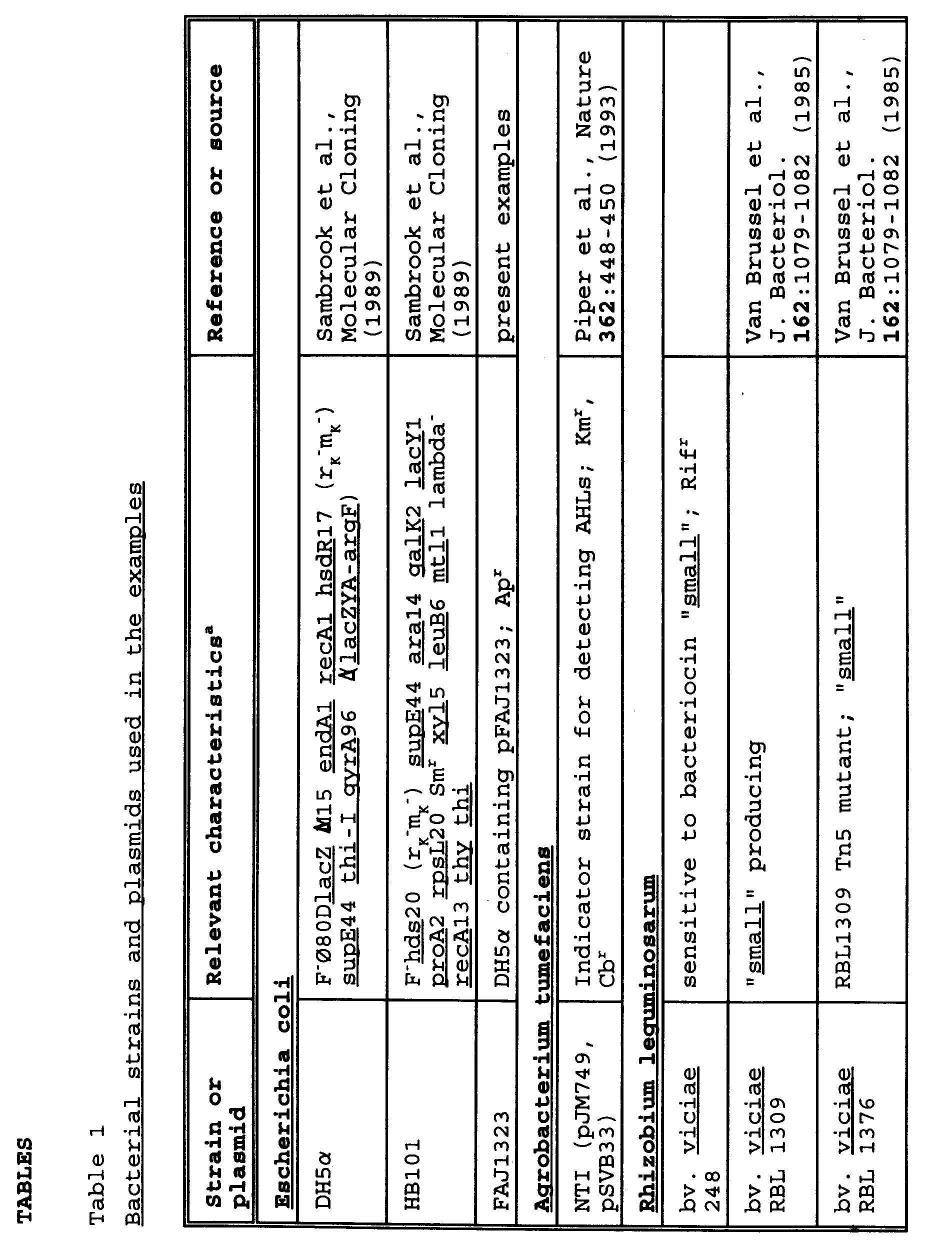

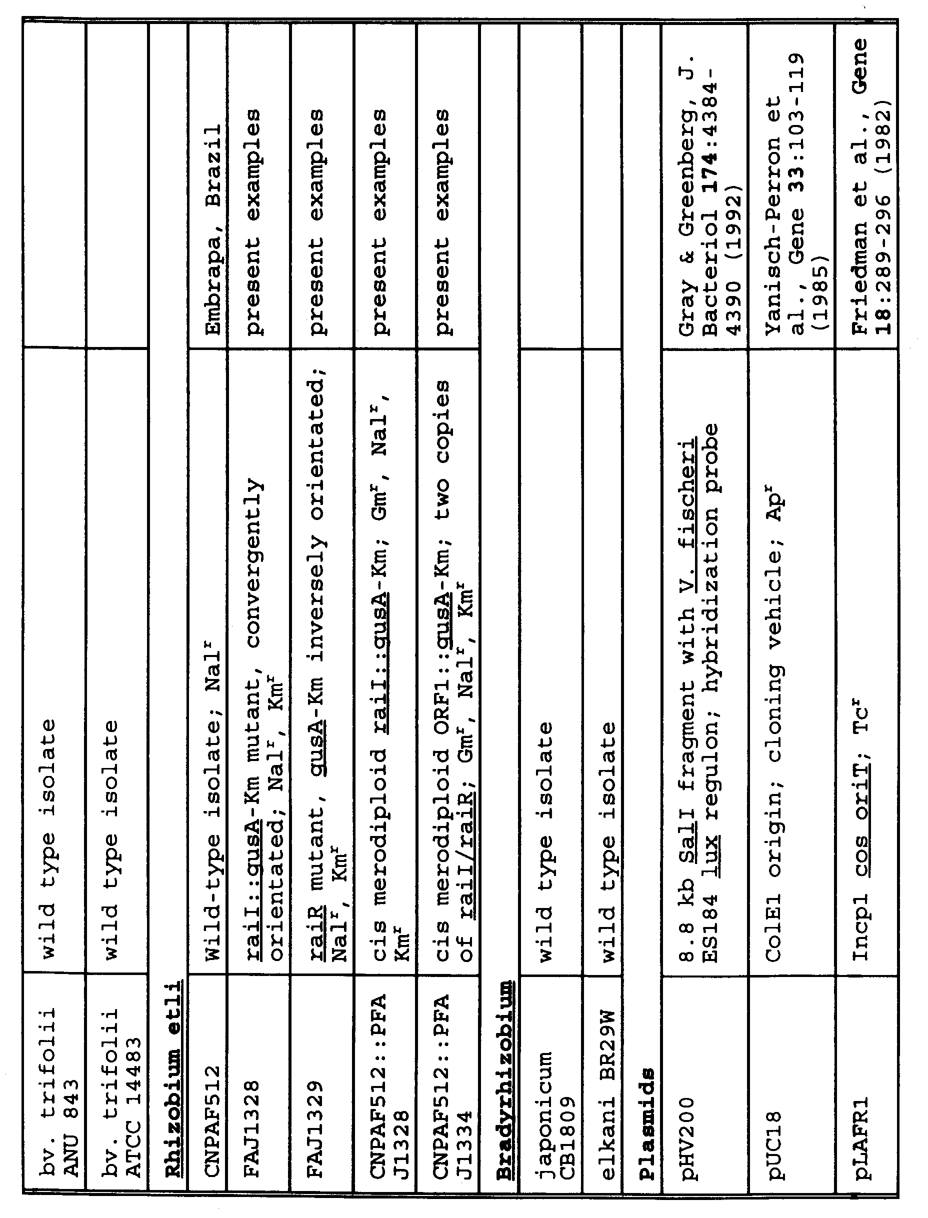

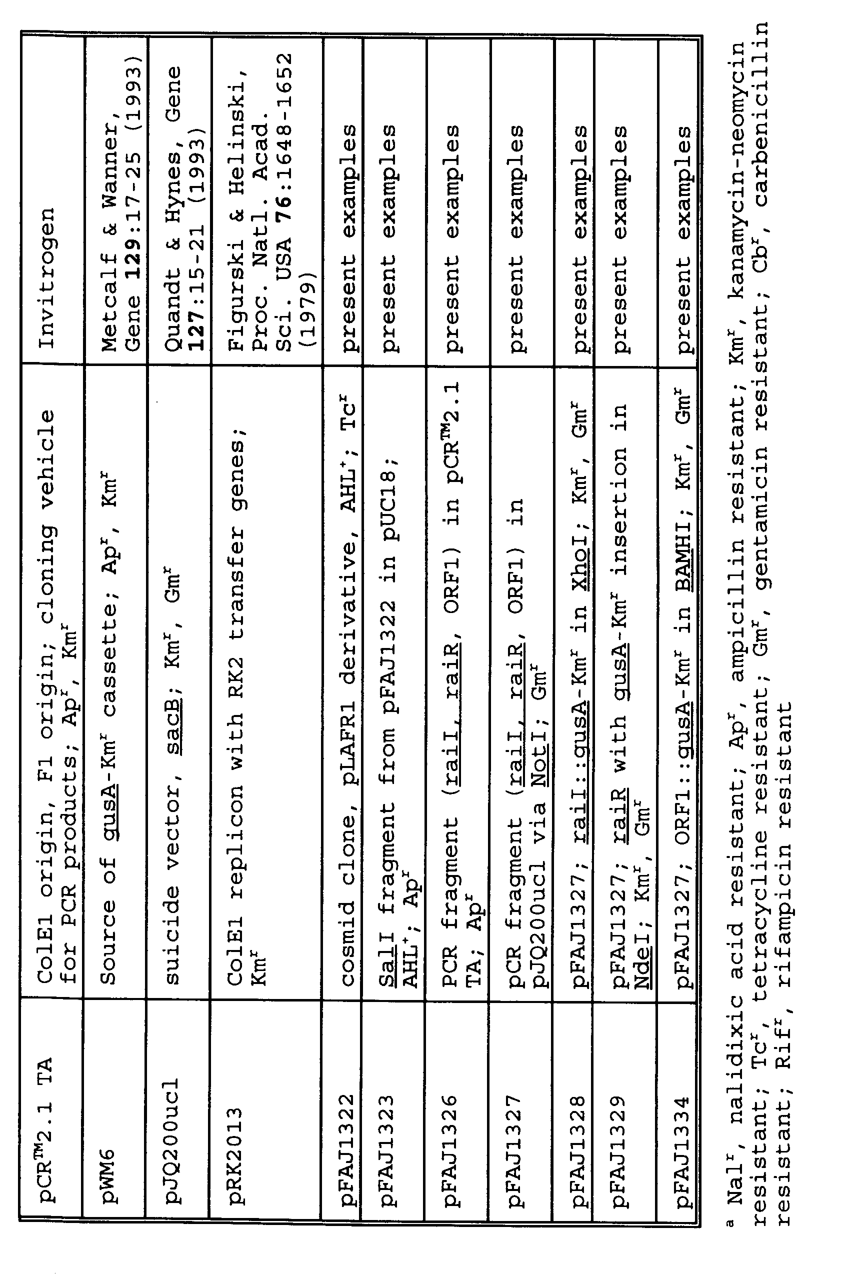

**Table 2**

| Identities and similarities of LuxI- and LuxR homologues^{a} | | | | |
|---|---|---|---|---|
| | Tra | Rhl | Lux | Rhi |
| RaiI | 28(47) | 25(40) | 23(40) | - |
| RaiR | 18(32) | 22(39) | 17(29) | 17(34) |

| | | | | |
|---|---|---|---|---|
| ^{a}The values for the number of identical or identical and similar (in brackets) amino acid residues are given as percentage referring to RaiI or RaiR. References for the sequences: Tral, A. tumefaciens (Hwang, J. et al., Proc. Natl. Acad. Sci. USA **91**:4639-4643 (1994)); RhlI and RhlR, Pseudomonas aeruginosa (Brint, J.M. & Ohman, D.E., J. Bacteriol **177**:7155-7163 (1995)); LuxI and LuxR, V. fischeri (Devine, J.H. et al.) Biochem. **27**:837-842 (1988)); TraR, A. tumefaciens (Piper, K.R. et al. Nature **362**:448-450 (1993)); RhiR, R. leguminosarum bv. viciae (Cubo, M.T. et al., J. Bacteriol. **174**:4026-4035 (1992)). | | | | |

**Table 3**

| Phenotypic characterization of mutants under symbiotic conditions^{a} | | | | |
|---|---|---|---|---|
| | CNPAF512 | FAJ1328 | FAJ1329 | CNPAF512: :pFAJ1334 |
| appearance of first nodules (d) | 9.1 (1.5)^{A} | 10.6 (1.8)^{A} | 10.0 (1.2)^{A} | 11.6 (1.6)^{A} |
| shoot dry weight (mg) | 217 (55)^{A} | 270 (63)^{A} | 218 (58)^{A} | 196 (45)^{A} |
| nodule number | 59.5 (15.3)^{A} | 118.4 (26.2)^{B} | 62.8 (17.4)^{A} | 44.9 (12.3)^{A} |
| nodule dry weight (mg) | 18.7 (5.6)^{A} | 31.6 (6.9)^{B} | 18.1 (4.9)^{A} | 10.9 (4.1)^{A} |
| acetylene reduction per plant (µmol h⁻¹) | 3.1 (0.4)^{A} | 5.1 (0.9)^{B} | 3.0 (0.4)^{A} | 1.9 (0.3)^{C} |
| acetylene reduction per nodule (µmol h⁻¹) | 52.1 (6.7)^{A} | 43.1 (7.6)^{A} | 47.8 (6.4)^{A} | 42.3 (6.7)^{A} |

| | | | | |
|---|---|---|---|---|
| ^{a}Values are based on examination of at least ten plants. The 95% confidence interval is given in brackets. Strains with the same letter are not significantly different for the tested parameter. | | | | |

## Claims

1. Mutant Rhizobium etli CNPAF512 strain, in which the biological function of the raiI gene is inactivated.

2. Mutant Rhizobium etli CNPAF512 strain as claimed in claim 1 having a gusA-Km^{r} insertion in its raiI gene.

3. Mutant Rhizobium etli CNPAF512 strain as claimed in claim 2 and herein designated as FAJ1328, having a gusA-Km^{r} insertion in the XhoI restriction site of the raiI gene.

4. Method for providing a Rhizobium etli CNPAF512 strain having a mutant raiI gene, comprising inactivation of the biological function of the raiI gene.

5. Method as claimed in claim 4, wherein the inactivation of the biological function of the raiI gene comprises insertion of a gusA-Km^{r} fragment in the XhoI restriction site of the raiI gene.

6. Method for providing a rhizobia strain having a mutant raiI gene, comprising:
a) identifying a gene that is similar to the raiI gene of Rhizobium etli CNPAF512 strain in any rhizobia strain; and
b) inactivating the biological function of the gene similar to raiI.

7. Method as claimed in claim 6, comprising:
a) identifying a gene that exhibits at least 40% sequence similarity with the raiI gene of Rhizobium etli CNPAF512 strain in any rhizobia strain; and
b) inactivating the biological function of the raiI homologous gene.

8. Method as claimed in claim 6, comprising:
a) identifying a gene that has a similar biological function as the raiI gene of Rhizobium etli CNPAF512 strain in any rhizobia strain; and
b) inactivating the biological function of the gene sharing sequence similarity with raiI.

9. Method as claimed in claim 8, wherein the biological function is production of one or more autoinducer synthases.

10. Method as claimed in claim 4 or 6-9, wherein the biological function of the raiI gene or the gene sharing sequence similarity with raiI or the gene having a biological function similar to raiI is inactivated by mutation.

11. Method as claimed in claim 10, wherein the mutation leads to either a disruption of the reading frame or an internal stop codon.

12. Method as claimed in claim 11, wherein the mutation is an insertion of any DNA sequence in the coding sequence of the gene, which insertion is capable of disrupting the reading frame.

13. Method as claimed in claim 11, wherein the mutation is a deletion of a part of the coding sequence of the gene and the deletion is capable of disrupting the reading frame.

14. Method as claimed in claim 4 or 6-9, wherein the biological function of the raiI gene or the gene sharing sequence similarity with raiI or the gene having a biological function similar to raiI is inactivated by interfering with the expression of one or more genes encoding transcription regulation factors involved in the transcription regulation of the raiI gene or the gene sharing sequence similarity with raiI or the gene having a biological function similar to raiI.

15. Rhizobia strain being deficient in the biological function of its raiI gene or the gene sharing sequence similarity with raiI or the gene having a biological function similar to raiI, obtainable by any one of the methods as claimed in claims 4 to 14.

16. Rhizobia strain as claimed in claim 15 for use in increasing the amount of nodules in their corresponding host plants.

17. Method for increasing the nitrogen fixation in Leguminosae, comprising inoculating Leguminosae plants with a mutant strain as claimed in claims 1, 2 or 3 or 12 or 13 and providing circumstances suitable for the rhizobia strain to induce root nodules.

18. Method as claimed in claim 17, wherein the Leguminosae plant is a bean plant and the rhizobia strain is a strain nodulating bean such as Rhizobium leguminosarum biovar phaseoli.

19. Method as claimed in claim 17, wherein the Leguminosae plant is a Phaseolus plant and the rhizobia strain is Rhizobium etli CNPAF512.

20. Method as claimed in claim 17, wherein the Leguminosae plant is a Glycine max plant and the rhizobia strain is a soy-bean nodulating strain selected from Bradyrhizobium japonicum CB1809 and Bradyrhizobium elkani BR29W.

21. raiI gene of Rhizobium etli CNPAF512 in substantially isolated form, comprising a coding nucleotide sequence that is identical to the coding sequence of the nucleotide sequence depicted in figure 1 or at least similar to this sequence, or the complementary sequence of either of these.

22. raiI gene as claimed in claim 21, wherein the similarity constitutes at least 90% sequence similarity.

23. raiI gene as claimed in claim 21, wherein the similarity constitutes a substantially same biological function.

24. raiI gene as claimed in claims 21-23 for use as a probe for screening other rhizobia strains for the presence of a gene that is similar to, in particular that shares at least 40% sequence similarity with or that has substantially the same biological function as raiI of Rhizobium etli CNPAF512.


SEQUENCE LISTING

(1) GENERAL INFORMATION:

(i) APPLICANT:
(A) NAME: K.U. Leuven Research & Development
(B) STREET: Groot Begijnhof/Benedenstraat 59
(C) CITY: LEUVEN
(E) COUNTRY: Belgium
(F) POSTAL CODE (ZIP): B-3000

(ii) TITLE OF INVENTION: Increase of nodule number and nitrogen
fixation in leguminosae

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98200498.8

(2) INFORMATION FOR SEQ ID NO: 1:

(i) SEQUENCE CHARACTERISTICS:
(A) LENGTH: 639 base pairs
(B) TYPE: nucleic acid
(C) STRANDEDNESS: double
(D) TOPOLOGY: linear

(ii) MOLECULE TYPE: DNA (genomic)

(vi) ORIGINAL SOURCE:
(A) ORGANISM: Rhizobium etli
(B) STRAIN: CNPAF512

(xi) SEQUENCE DESCRIPTION: SEQ ID NO: 1:

ATGTTGCGGA TACTCACCAA AGACATGCTC GAGACCGATC GACGGGCTTT CGATGAAATG 60

TTTCGGGCTC GCGCCGCCGT TTTTCGAGAT CGTCTGGGAT GGCAGGTCGA TGTCCGGGAT 120

CAATGGGAGA GGGACCGATA CGACGAGGCT GAGGATCCCG TCTATCTCGT CACGCAACAA 180

CCTTCCGGCA CGCTGACGGG TTCGCTGCGC CTGCTGCCGA CCACCGGCGC GACGATGCTC 240

AAAAGCGAGT TCCGGCATTT CTTCGATCAG CCTATCGACG TTGATAGCCC GACGACCTGG 300

GAATGTACCC GCTTTTGCCT TCATCCGCAT GCCGGCGATA TGAAGCAATC GCGCGCAGTC 360

GCCACGGAGC TGCTCTCAGG GCTTTGCGAT CTTGCGCTCG ACACCGGCAT CGAAAACATT 420

GTCGGCGTCT ATGACGTCGC GATGGTCGCG GTGTACCGGA GGATCGGCTG GAGGCCGACG 480

CCGCTTGCCC GATCCCGGCC CGAGATCGGC AAGCTGTATG TTGGTCTATG GGATGTGACG 540

GCGGACAATT GTCGGACACT TAGGGCCAAC CTGTCCCGAC TTCTGGAGCA AGCCTCTCCC 600

TATCCTGCCA GAGTCCTTGT CGATGGCGGC ATGCGATAG 639

(2) INFORMATION FOR SEQ ID NO: 2:

(i) SEQUENCE CHARACTERISTICS:
(A) LENGTH: 212 amino acids
(B) TYPE: amino acid
(C) STRANDEDNESS:
(D) TOPOLOGY: unknown

(ii) MOLECULE TYPE: protein

(vi) ORIGINAL SOURCE:
(A) ORGANISM: Rhizobium etli
(B) STRAIN: CNPAF512

(xi) SEQUENCE DESCRIPTION: SEQ ID NO: 2:

Met Leu Arg Ile Leu Thr Lys Asp Met Leu Glu Thr Asp Arg Arg Ala
1               5                   10                  15

Phe Asp Glu Met Phe Arg Ala Arg Ala Ala Val Phe Arg Asp Arg Leu
            20                  25                  30

Gly Trp Gln Val Asp Val Arg Asp Gln Trp Glu Arg Asp Arg Tyr Asp
        35                  40                  45

Glu Ala Glu Asp Pro Val Tyr Leu Val Thr Gln Gln Pro Ser Gly Thr
    50                  55                  60

Leu Thr Gly Ser Leu Arg Leu Leu Pro Thr Thr Gly Ala Thr Met Leu
65                  70                  75                  80

Lys Ser Glu Phe Arg His Phe Phe Asp Gln Pro Ile Asp Val Asp Ser
                85                  90                  95

Pro Thr Thr Trp Glu Cys Thr Arg Phe Cys Leu His Pro His Ala Gly
            100                 105                 110

Asp Met Lys Gln Ser Arg Ala Val Ala Thr Glu Leu Leu Ser Gly Leu
        115                 120                 125

Cys Asp Leu Ala Leu Asp Thr Gly Ile Glu Asn Ile Val Gly Val Tyr
    130                 135                 140

Asp Val Ala Met Val Ala Val Tyr Arg Arg Ile Gly Trp Arg Pro Thr
145                 150                 155                 160

Pro Leu Ala Arg Ser Arg Pro Glu Ile Gly Lys Leu Tyr Val Gly Leu
                165                 170                 175

Trp Asp Val Thr Ala Asp Asn Cys Arg Thr Leu Arg Ala Asn Leu Ser
            180                 185                 190

Arg Leu Leu Glu Gln Ala Ser Pro Tyr Pro Ala Arg Val Leu Val Asp
        195                 200                 205

Gly Gly Met Arg
    210

(2) INFORMATION FOR SEQ ID NO: 3:

    (i) SEQUENCE CHARACTERISTICS:
        (A) LENGTH: 212 amino acids
        (B) TYPE: amino acid
        (C) STRANDEDNESS:
        (D) TOPOLOGY: unknown

    (ii) MOLECULE TYPE: protein

    (vi) ORIGINAL SOURCE:
        (A) ORGANISM: Rhizobium etli
        (B) STRAIN: CNPAF512

    (xi) SEQUENCE DESCRIPTION: SEQ ID NO: 3:

Met Leu Arg Ile Leu Thr Lys Asp Met Leu Glu Thr Asp Arg Arg Ala
1               5                   10                  15

Phe Asp Glu Met Phe Arg Ala Arg Ala Ala Val Phe Arg Asp Arg Leu
            20                  25                  30

Gly Trp Gln Val Asp Val Arg Asp Gln Trp Glu Arg Asp Arg Tyr Asp
        35                  40                  45

Glu Ala Glu Asp Pro Val Tyr Leu Val Thr Gln Gln Pro Ser Gly Thr
    50                  55                  60

Leu Thr Gly Ser Leu Arg Leu Leu Pro Thr Thr Gly Ala Thr Met Leu
65                  70                  75                  80

Lys Ser Glu Phe Arg His Phe Phe Asp Gln Pro Ile Asp Val Asp Ser
                85                  90                  95

Pro Thr Thr Trp Glu Cys Thr Arg Phe Cys Leu His Pro His Ala Gly
            100                 105                 110

Asp Met Lys Gln Ser Arg Ala Val Ala Thr Glu Leu Leu Ser Gly Leu
        115                 120                 125

Cys Asp Leu Ala Leu Asp Thr Gly Ile Glu Asn Ile Val Gly Val Tyr
    130                 135                 140

Asp Val Ala Met Val Ala Val Tyr Arg Arg Ile Gly Trp Arg Pro Thr
145                 150                 155                 160

Pro Leu Ala Arg Ser Arg Pro Glu Ile Gly Lys Leu Tyr Val Gly Leu
                165                 170                 175

Trp Asp Val Thr Ala Asp Asn Cys Arg Thr Leu Arg Ala Asn Leu Ser
            180                 185                 190

Arg Leu Leu Glu Gln Ala Ser Pro Tyr Pro Ala Arg Val Leu Val Asp
        195                 200                 205

Gly Gly Met Arg
    210

(2) INFORMATION FOR SEQ ID NO: 4:

(i) SEQUENCE CHARACTERISTICS:
(A) LENGTH: 193 amino acids
(B) TYPE: amino acid
(C) STRANDEDNESS:
(D) TOPOLOGY: unknown

(ii) MOLECULE TYPE: protein

(vi) ORIGINAL SOURCE:
(A) ORGANISM: Vibrio fischeri

(xi) SEQUENCE DESCRIPTION: SEQ ID NO: 4:

Met Thr Ile Met Ile Lys Lys Ser Asp Phe Leu Ala Ile Pro Ser Glu
1               5                   10                  15

Glu Tyr Lys Gly Ile Leu Ser Leu Arg Tyr Gln Val Phe Lys Gln Arg
            20                  25                  30

Leu Glu Trp Asp Leu Val Val Glu Asn Asn Leu Glu Ser Asp Glu Tyr
        35                  40                  45

Asp Asn Ser Asn Ala Glu Tyr Ile Tyr Ala Cys Asp Asp Thr Glu Asn
    50                  55                  60

Val Ser Gly Cys Trp Arg Leu Leu Pro Thr Thr Gly Asp Tyr Met Leu
65                  70                  75                  80

Lys Ser Val Phe Pro Glu Leu Leu Gly Gln Gln Ser Ala Pro Lys Asp
                85                  90                  95

Pro Asn Ile Val Glu Leu Ser Arg Phe Ala Val Gly Lys Asn Ser Ser
            100                 105                 110

Lys Ile Asn Asn Ser Ala Ser Glu Ile Thr Met Lys Leu Phe Glu Ala
        115                 120                 125

Ile Tyr Lys His Ala Val Ser Gln Gly Ile Thr Glu Tyr Val Thr Val
    130                 135                 140

Thr Ser Thr Ala Ile Glu Arg Phe Leu Lys Arg Ile Lys Val Pro Cys
145                 150                 155                 160

His Arg Ile Gly Asp Lys Glu Ile His Val Leu Gly Asp Thr Lys Ser
                165                 170                 175

Val Val Leu Ser Met Pro Ile Asn Glu Gln Phe Lys Lys Ala Val Leu
            180                 185                 190

Asn

(2) INFORMATION FOR SEQ ID NO: 5:

    (i) SEQUENCE CHARACTERISTICS:
        (A) LENGTH: 211 amino acids
        (B) TYPE: amino acid
        (C) STRANDEDNESS:
        (D) TOPOLOGY: unknown

    (ii) MOLECULE TYPE: protein

    (vi) ORIGINAL SOURCE:
        (A) ORGANISM: Agrobacterium tumefaciens

    (xi) SEQUENCE DESCRIPTION: SEQ ID NO: 5:

Met Arg Ile Leu Thr Val Ser Pro Asp Gln Tyr Glu Arg Tyr Arg Ser
1               5                   10                  15

Phe Leu Lys Gln Met His Arg Leu Arg Ala Thr Val Phe Gly Gly Arg
            20                  25                  30

Leu Glu Trp Asp Val Ser Ile Ile Ala Gly Glu Glu Arg Asp Gln Tyr
        35                  40                  45

Asp Asn Phe Lys Pro Ser Tyr Leu Leu Ala Ile Thr Asp Ser Gly Arg
    50                  55                  60

Val Ala Gly Cys Val Arg Leu Leu Pro Ala Cys Gly Pro Thr Met Leu
65                  70                  75                  80

Glu Gln Thr Phe Ser Gln Leu Leu Glu Met Gly Ser Leu Ala Ala His
                85                  90                  95

Ser Gly Met Val Glu Ser Ser Arg Phe Cys Val Asp Thr Ser Leu Val
            100                 105                 110

Ser Arg Arg Asp Ala Ser Gln Leu His Leu Ala Thr Leu Thr Leu Phe
        115                 120                 125

Ala Gly Ile Ile Glu Trp Ser Met Ala Ser Gly Tyr Thr Glu Ile Val
    130                 135                 140

Thr Ala Thr Asp Leu Arg Phe Glu Arg Ile Leu Lys Arg Ala Gly Trp
145                 150                 155                 160

Pro Met Arg Arg Leu Gly Glu Pro Thr Ala Ile Gly Asn Thr Ile Ala
                165                 170                 175

Ile Ala Gly Arg Leu Pro Ala Asp Arg Ala Ser Phe Glu Gln Val Cys
            180                 185                 190

Pro Pro Gly Tyr Tyr Ser Ile Pro Arg Ile Asp Val Ala Ala Ile Arg
        195                 200                 205

Ser Ala Ala
    210

(2) INFORMATION FOR SEQ ID NO: 6:

    (i) SEQUENCE CHARACTERISTICS:
        (A) LENGTH: 196 amino acids
        (B) TYPE: amino acid
        (C) STRANDEDNESS:
        (D) TOPOLOGY: unknown

    (ii) MOLECULE TYPE: protein

    (vi) ORIGINAL SOURCE:
        (A) ORGANISM: Pseudomonas aeruginosa

    (xi) SEQUENCE DESCRIPTION: SEQ ID NO: 6:

Met Ile Glu Leu Leu Ser Glu Ser Leu Glu Gly Leu Ser Ala Ala Met
1               5                   10                  15

Ile Ala Glu Leu Gly Arg Tyr Arg His Gln Val Phe Ile Glu Lys Leu
            20                  25                  30

Gly Trp Asp Val Val Ser Thr Ser Arg Val Arg Asp Gln Glu Phe Asp
        35                  40                  45

Gln Phe Asp His Pro Gln Thr Arg Tyr Ile Val Ala Met Ser Arg Gln
    50                  55                  60

Gly Ile Cys Gly Cys Ala Arg Leu Leu Pro Thr Thr Asp Ala Tyr Leu
65                  70                  75                  80

Leu Lys Asp Val Phe Ala Tyr Leu Cys Ser Glu Thr Pro Pro Ser Asp
                85                  90                  95

Pro Ser Val Trp Glu Leu Ser Arg Tyr Ala Ala Ser Ala Ala Asp Asp
            100                 105                 110

Pro Gln Leu Ala Met Lys Ile Phe Trp Ser Ser Leu Gln Cys Ala Trp
        115                 120                 125

Tyr Leu Gly Ala Ser Ser Val Val Ala Val Thr Thr Thr Ala Met Glu
    130                 135                 140

Arg Tyr Phe Val Arg Asn Gly Val Ile Leu Gln Arg Leu Gly Pro Pro
145                 150                 155                 160

Gln Lys Val Lys Gly Glu Thr Leu Val Ala Ile Ser Phe Pro Ala Tyr
                165                 170                 175

Gln Glu Arg Gly Leu Glu Met Leu Leu Arg Tyr His Pro Glu Trp Leu
            180                 185                 190

Ala Glu Pro Arg
        195